# EUROPÄISCHE PATENTSCHRIFT

(11) **EP 3 169 768 B1**
(45) Veröffentlichungstag und Bekanntmachung des Hinweises auf die Patenterteilung: **26.09.2018**
(21) Anmeldenummer: 15744499.3
(22) Anmeldetag: 10.07.2015
(51) Int. Cl.: C12N 1/18, C12N 1/32, C12N 9/04, C12N 9/12, C12P 7/04, C12P 7/06, C12P 7/18, C12P 7/46

(54) **GENTECHNISCH VERÄNDERTE HEFE MIT VERBESSERTEM GLYCEROL-KATABOLISMUS**
GENETICALLY MODIFIED YEAST WITH IMPROVED GLYCEROL CATABOLISM
LEVURE GÉNÉTIQUEMENT MODIFIÉE PRÉSENTANT UN CATABOLISME DU GLYCÉROL AMÉLIORÉ

(30) Priorität: 14.07.2014 DE 102014109858; 11.11.2014 EP 14192729
(43) Veröffentlichungstag der Anmeldung: 24.05.2017
(73) Patentinhaber: Jacobs University Bremen GmbH, 28759 Bremen (DE)
(72) Erfinder: NEVOIGT, Elke, 10439 Berlin (DE)
(74) Vertreter: Weidner Stern Jeschke
(86) Internationale Anmeldenummer: PCT/EP2015/065839
(87) Internationale Veröffentlichungsnummer: WO 2016/008819

(56) Entgegenhaltungen:
- EP-A1- 2 113 570
- WO-A1-2010/019882
- WO-A1-2011/022651
- WO-A1-2011/149353
- WO-A1-2012/067510
- WO-A2-2013/081456
- WO-A2-2014/074895
- WO-A2-2015/028583
- US-A1- 2013 109 070
- US-A1- 2013 149 754
- IRSHAD AHMAD ET AL: "Enhancement of xylitol production in glycerol kinase disrupted Candida tropicalis by co-expression of three genes involved in glycerol metabolic pathway", BIOPROCESS AND BIOSYSTEMS ENGINEERING, Bd. 36, Nr. 9, 12. Dezember 2012 (2012-12-12), Seiten 1279-1284, XP55214813, ISSN: 1615-7591, DOI: 10.1007/s00449-012-0872-4
- STEVE SWINNEN ET AL: "Re-evaluation of glycerol utilization in Saccharomyces cerevisiae: characterization of an isolate that grows on glycerol without supporting supplements", BIOTECHNOLOGY FOR BIOFUELS, BIOMED CENTRAL LTD, GB, Bd. 6, Nr. 1, 8. November 2013 (2013-11-08), Seite 157, XP021168764, ISSN: 1754-6834, DOI: 10.1186/1754-6834-6-157
- YU K O ET AL: "Engineering of glycerol utilization pathway for ethanol production by Saccharomyces cerevisiae", BIORESOURCE TECHNOLOGY, ELSEVIER BV, GB, Bd. 101, Nr. 11, 9. Februar 2010 (2010-02-09), Seiten 4157-4161, XP026917458, ISSN: 0960-8524, DOI: 10.1016/J.BIORTECH.2010.01.066 [gefunden am 2010-02-09]
- NEVES L ET AL: "New insights on glycerol transport in Saccharomyces cerevisiae", FEBS LETTERS, ELSEVIER, AMSTERDAM, NL, Bd. 565, Nr. 1-3, 9. April 2004 (2004-04-09), Seiten 160-162, XP004507697, ISSN: 0014-5793, DOI: 10.1016/J.FEBSLET.2004.04.003

## Beschreibung

Die Erfindung betrifft eine gentechnisch veränderte Hefe mit verbessertem Glycerol-Katabolismus.

Die Herstellung von Biokraftstoffen und Chemikalien aus erneuerbaren Ressourcen ist notwendig, um den Energiebedarf in einer Welt zu decken, in der mineralölbasierte Kraftstoffe immer knapper und teurer werden. Biodiesel ist ein alternativer Kraftstoff, der in Deutschland derzeit größtenteils aus Raps gewonnen wird und nach einer Umfrage des Verbands der Deutschen Biokraftstoffindustrie e.V. die Treibhausgasemission im Vergleich zu fossilem Diesel bis zu 60% reduzieren kann. Prinzipiell können auch Altspeisefette verwendet werden. Die potentielle Nutzung von Fetten bzw. Ölen, die durch Mikroalgen bzw. anderen Mikroorganismen produziert werden (Gong and Jiang, 2011; Shi et al., 2011), sind in der Diskussion, spielen aber kommerziell bisher keine Rolle. Biodiesel wird hauptsächlich hergestellt durch die Umesterung von Fetten und pflanzlichen Ölen in Gegenwart eines Alkohols (meist Methanol), was zur Bildung eines Fettsäuremethylesters (Biodiesel) und Glycerol (als Nebenprodukt) führt. Die Biodieselproduktion und damit auch die Menge an Rohglycerol, die parallel erzeugt wird, hat in den letzten Jahren stark zugenommen. Pro 100 Gewichtseinheiten hergestelltem Biodiesel fallen 10 Gewichtseinheiten Rohglycerol an (Yazdani & Gonzalez, 2007). In Deutschland wurden im Jahr 2011 200.000 t Rohglycerol im Zuge der Biodieselherstellung produziert (Marktanalyse Nachwachsende Rohstoffe, FNR, Schriftenreihe Nachwachsende Rohstoffe 34, 2014). Die Aufreinigung der Glycerolfraktion aus Rohglycerol in einer Weise, dass es die Standards der Lebensmittel-, Pharma- oder Kosmetikindustrie erfüllt, ist nicht wirtschaftlich, so dass andere Wege beschritten werden müssen, um Rohglycerol zu verwerten. Eine Möglichkeit ist die Verwendung als Ausgangsmaterial für mikrobielle Umwandlungsverfahren (Almeida et al., 2012).

Die Bäckerhefe (*Saccharomyces cerevisiae*) ist ein beliebtes Objekt des Metabolic Engineering sowie ein attraktiver Produktionsorganismus in der industriellen Biotechnologie. Es wurden zahlreiche Ansätze verfolgt, um diesen Organismus zur Herstellung von industriell relevanten Produkten wie Biokraftstoffen, Grund- und Feinchemikalien (einschließlich Arzneimitteln) sowie Proteinwirkstoffen nutzbar zu machen (Borodina & Nielsen, 2014; Hong & Nielsen, 2012; Nevoigt, 2008). *S*. *cerevisiae* wächst jedoch nur schlecht auf Glycerol als Kohlenstoffquelle. Zwei Studien haben gezeigt, dass die häufig in der Forschung und industriell verwendeten *S*.-*cerevisiae-*Stämme die Zugabe von Ergänzungsstoffen (wie Aminosäuren und Nukleobasen) erfordern, um auf Glycerol zu wachsen (Merico et al 2011; Swinnen et al., 2013). Lediglich einzelne Isolate sind in der Lage, Glycerol ohne solche Medienzusätze als Kohlenstoffquelle zu nutzen (Swinnen et al. 2013).

Zur Metabolisierung muss Glycerol zunächst in die Zelle aufgenommen werden. Bei Mikroorganismen sind grundsätzlich zwei Arten von Transportsystemen gefunden worden (s. Fakas et al. 2009 und Lin et al. 1976). Der erste Typ beruht auf erleichterter Diffusion, benötigt keine Energie und hängt von Glycerol-Facilitatoren ab. Im Gegensatz dazu ist der zweite Typ ein energieabhängiger aktiver Transport auf Basis eines Glycerol/H⁺-Symporters. Es war lange umstritten, wie Glycerol in den *S*. *cerevisiae* Zelle transportiert wird (Neves et al. 2004). Schließlich hat sich gezeigt, dass ein durch *STL1* codiertes aktives Transportsystem von entscheidender Bedeutung ist (Ferreira et al. 2005). Ein von *FPS1* codierter Glycerol-Facilitator ist natürlichweise auch in *S*. *cerevisiae* vorhanden, scheint bei dieser Art aber keine entscheidende Rolle für die Glycerol-Aufnahme zu spielen.

Generell erfolgt der initiale Abbau von Glycerol in Mikroorganismen auf drei Wegen (s. Fakas et al. 2009). Der Glycerol-3-Phosphat-Weg (auch G3P-Weg oder Phosphorylierungsweg) führt über die Phosphorylierung von Glycerol durch die Glycerolkinase zu L-Glycerol-3-phosphat (G3P), welches durch die Glycerol-3-phosphat-Dehydrogenase zu Dihydroxyacetonphosphat (DHAP) oxidiert wird. Der DHA-Weg (auch Oxidationsweg) führt über die durch Glyceroldehydrogenase katalysierte Oxidation von Glycerol zu Dihydroxyaceton (DHA), welches von der Dihydroxacetonkinase zu Dihydroxyacetonphosphat (DHAP) phosphoryliert wird. Der Glycerinaldehyd-Weg führt über Glycerinaldehyd zu Glycerinaldeyd-3-Phosphat, das enzymatisch zu DHAP umgewandelt werden kann.

Experimentelle Daten deuten darauf hin, dass *S*. *cerevisiae* Glycerol ausschließlich über den Phosphorylierungsweg, d.h. über L-Glycerin-3-phosphat, abbaut, d.h. lediglich die von *GUT1* und *GUT2* codierten Enzyme Glycerolkinase und Glycerol-3-Phosphat-Dehydrogenase verwendet. Experimentelle Beweise für die Bedeutung von Gutl und Gut2 für das Wachstum von *S*. *cerevisiae* auf Glycerol wurden erstmalig von Sprague und Cronan (1977) bereitgestellt.

Die Studien von Ferreira et al. (2005) zur Bedeutung des Stl1 Transporters bzw. von Sprague und Cronan (1977) zur Rolle von Gut1 und Gut2 für die Glycerolverwertung von *S*. *cerevisiae* wurden in Medien durchgeführt, die komplexe Zusätze wie mehrere Aminosäuren und Nukleobasen enthielt. Swinnen et al. 2013 konnten allerdings zeigen, dass die Gene *STL1*, *GUT1* und *GUT2* auch eine entscheidende und exklusive Rolle für das Wachstum von *S*. *cerevisiae* in Glycerol-haltigem Medium ohne Zusätze haben.

Der oxidative Glycerol-Abbauweg über DHA scheint bei *Neurospora crassa* parallel zum Phosphorylierungsweg vorhanden zu sein (Tom et al., 1978). Eine NAD⁺-abhängige Glyceroldehydrogenase wurde in einigen Arten der Gattungen und *Candida*, *Torulopsis* und *Hansenula* gefunden (Hofmann und Babel, 1979); später auch in *Schizosaccharomyces pombe* (May & Sloan, 1981). Babel und Hofmann (1982) konnten bei *Candida valida* eine besonders hohe Aktivität dieses Enzyms messen. Es ist allerdings unklar, ob der oxidative Weg in *S. cerevisiae* natürlicherweise funktional ist. In der Tat zeigten weder eine *GUT1*- noch eine *GUT2*-Deletionsmutante signifikantes Wachstum auf Glycerol (Swinnen et al. 2013), ein Ergebnis, das eine wichtige Rolle des DHA-Weges für diese Funktion ausschließt. Darüber hinaus konnten weder Norbeck und Blomberg (1997) noch Nguyen und Nevoigt (2009) eine native Glycerol-Dehydrogenase-Aktivität in *S*. *cerevisiae* messen. Im Gegensatz dazu schlagen andere Autoren vor, dass Glycerol durch eine NADP⁺-abhängige Glycerin-Dehydrogenase (GDH) zu Dihydroxyaceton umgewandelt wird, wobei die Gene *ARA1*, *GCY1*, *GRE3* und *YPR1* beteiligt sein sollen (Izawa et al., 2004). Zwar gibt es Zweifel an der Existenz einer natürlichen Glyceroldehydrogenase in *S*. *cerevisiae*, eine messbare Aktivität des zweiten Enzyms des DHA-Wegs, der durch die Gene und *DAK1* und *DAK2* codierten Dihydroxyacetonkinase, ist in *S*. *cerevisiae* jedoch natürlicherweise vorhanden (Molin et al., 2003). Eine Studie von Jung et al. 2011 hat kürzlich im Übrigen gezeigt, dass die Expression einer heterologen Glycerol-Dehydrogenase aus *Ogataea angusta* (Syn. *Pichia angusta*, *Hansenula polymorpha*) in *S. cerevisiae* die Glycerol-Nutzung im Vergleich zum Wildtyp-Stamm 1,22-fach verbesserte, d.h. der gentechnisch veränderte Stamm verbrauchte nach 96 h Kultivierung 5,18 g/l von 12,62 g/l Glycerol, während die Kontrolle im Vergleich dazu in der gleichen Zeit nur 4,24 g/l verbrauchte.

Die WO 2012/067510 A1 beschreibt die Modifikation einer Hefezelle durch Aktivitätserhöhung einer NAD-abhängigen Glyceroldehydrogenase und einer Dihydroxyacetonkinase.

In dem Artikel "Enhancement of yxlitol production in glycerol kinase disrupted Candida tropicalis by co-expression of three genes involved in glycerol metabolic pathway" von Isshad Ahmad, Woo Yong Shim und Jung-Hoe Kim in "Bioprocess Biosyst Eng" (2013) 36: 1279-1284 wird eine Blockade des G3P-Weges in einer Hefezelle durch Ausschaltung von GUT1 (Glycerolkinase, gk) beschrieben.

Aufgabe der vorliegenden Erfindung ist es, die Fähigkeit von Hefe, Glycerol als Kohlenstoffquelle zu nutzen, zu verbessern.

Gelöst wird die Aufgabe durch eine gentechnisch veränderte Hefezelle der Gattung *Saccharomyces* mit verbessertem Glycerol-Katabolismus, wobei die Hefezelle dahingehend gentechnisch verändert ist, dass
a) der Abbau von Glycerol zu Dihydroxyacetonphosphat über den Glycerol-3-Phosphat-Weg blockiert ist,
b) ein heterologes Glycerolaufnahme-Facilitator-Protein exprimiert wird, und
c) eine die Oxidation von Glycerol zu Dihydroxyaceton katalysierende heterologe NAD-abhängige Glyceroldehydrogenase exprimiert oder eine die Oxidation von Glycerol zu Dihydroxyaceton katalysierende heterologe NADH-abhängige Glyceroldehydrogenase überexprimiert wird, und
f) eine heterologe Dihydroxyacetonkinase exprimiert oder die native Dihydroxyacetonkinase überexprimiert wird.

Die erfindungsgemäß vorgesehene Kombination von gentechnischen Veränderungen an *Saccharomyces*-Zellen bewirkt eine effiziente Aufnahme und Kanalisierung von Glycerol durch den nunmehr etablierten oder zumindest verstärkten DHA-Stoffwechselweg. Beschrieben werden außerdem Zellen, in denen der Glycerolkatabolismus über den Glycerinaldehyd-Weg etabliert oder optimiert, indem mindestens eines, vorzugsweise sämtliche der an diesem Weg bis hin zum Glycerinaldehyd-3-Phosphat beteiligten natürlichen (nativen) Enzyme überexprimiert oder durch ein heterologes Enzym mit der gleichen Enzymaktivität ersetzt wird. In diesen Zellen ist es nicht erforderlich und auch nicht bevorzugt, den DHA-Weg zu manipulieren. Die Integration eines heterologen Glycerol-Facilitators fördert die Aufnahme von Glycerol, ohne dass hierfür Energie benötigt wird. Die Blockade des Glycerol-3-phosphat-Wegs führt dazu, dass in die Zelle aufgenommenes Glycerol entweder über den DHA-Weg abgebaut wird, wobei durch die Einführung einer heterologen Glyceroldehydrogenase oder die Überexpression eines nativen Enzyms mit Glyceroldehydrogenaseaktivität der etwaige Mangel an einer natürlichen Glyceroldehydrogenaseaktivität kompensiert ist, oder alternativ über den dann durch Überexpression der beteiligten Enzyme oder Einführung heterologer Enzymvarianten optimierten Glycerinaldehyd-Weg. Die am Glycerol-3-Phosphat-Weg beteiligte FADabhängige Glycerol-3-phosphat-Dehydrogenase ist auf der Außenseite der der inneren Mitochondrienmembran lokalisiert (Gancedo et al. 1968; Klingenberg 1970) und leitet Elektronen aus der Oxidation von Glycerol-3-phosphat direkt in die mitochondriale Atmungskette, was nachteilig ist, wenn Reduktionsäquivalente beispielsweise für eine nachfolgende Herstellung reduzierter Verbindungen, beispielsweise 1,2-Propandiol, benötigt werden. Demgegenüber liefert der DHA-Weg wertvolle Reduktionsäquivalente in Form von NADH, der Glycerinaldehyd-Weg in Form von NADPH.

Unter dem Begriff "Glycerol-3-Phosphat-Weg", "Glycerol-3-Phosphat-Stoffwechselweg", "G3P-Weg" oder auch "Phosphorylierungsweg" wird die Umsetzung von Glycerol über L-Glycerol-3-phosphat (G3P) zu Dihydroxacetonphosphat (DHAP) verstanden.

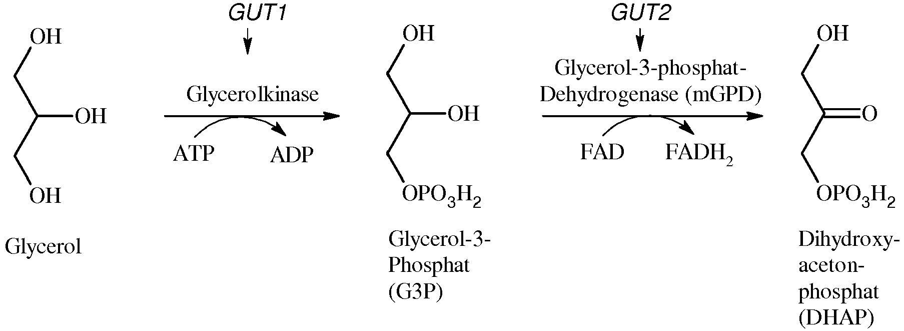

Die Reaktionen werden von der Glycerolkinase und der mitochondrialen (FAD-abhängigen) Glycerol-3-phosphat-Dehydrogenase (GPD-M, mGPD, EC 1.1.5.3) katalysiert. In der Hefe *Saccharomyces cerevisiae* wird die Glycerolkinase durch das Gen *GUT1*, die Glycerol-3-phosphat-Dehydrogenase durch *GUT2* kodiert.

Unter dem "DHA-Weg", "DHA-Stoffwechselweg" oder "Oxidationsweg" wird die Umsetzung von Glycerol über Dihydroxyaceton (DHA) zu Dihydroxacetonphosphat (DHAP) verstanden.

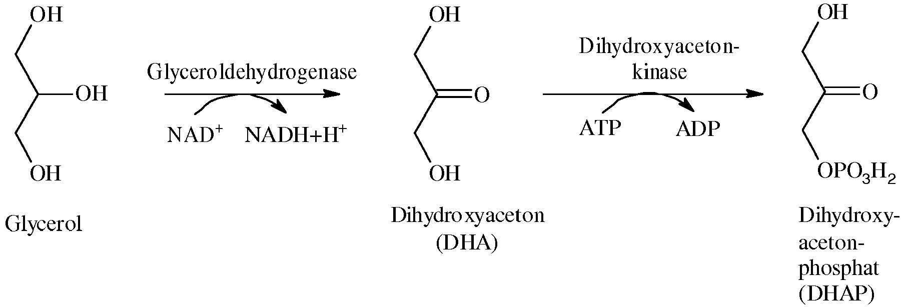

Die Reaktionen werden von der Glyceroldehydrogenase und der Dihydroxyacetonkinase katalysiert.

Unter dem Begriff "Glycerinaldehyd-Weg" wird die Umsetzung von Glycerol über D-Glycerinaldehyd zu D-Glycerinaldehyd-3-Phosphat (GAP)
verstanden.

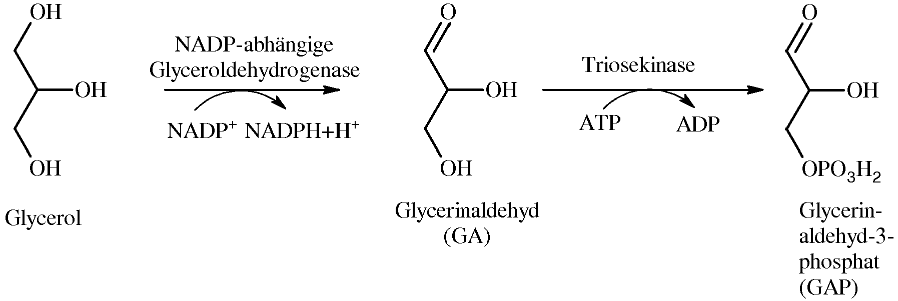

Die Reaktionen werden von einer NADP-abhängigen Glyceroldehydrogenase (NADP-Glyceroldehydrogenase, NADP-GDH) und einer Glycerinaldehyd-Kinase (Triokinase, Triosekinase, EC 2.7.1.28) katalysiert.

Wenn hier von einem "Syntheseweg zur Rückbildung von Glycerol aus Dihydroxyacetonphosphat" gesprochen wird, ist damit die Umsetzung von Dihydroxyacetonphosphat über Glycerol-3-phosphat zu Glycerol gemeint.

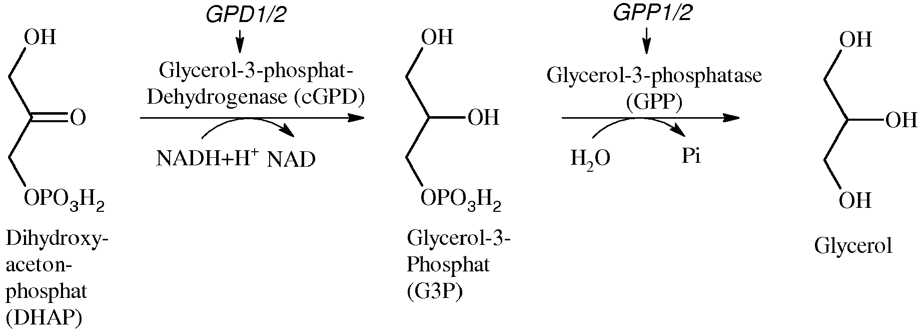

Die Reduktion von DHAP zu Glycerol-3-phosphat wird durch die in zwei Isoformen vorkommende cytosolische NAD-abhängige Glycerol-3-phosphat-Dehydrogenase (GPD-C, cGPD, EC 1.1.1.8) katalysiert. Die Isoformen werden von den Genen *GPD1* bzw. *GPD2* kodiert. Die Dephosphorylierung von Glycerol-3-phosphat zu Glycerol wird von der Glycerol-3-phosphatase (GPP, EC 3.1.3.21) katalysiert, die ebenfalls in zwei Isoformen vorkommt, die von *GPP1* und *GPP2* kodiert werden.

Der Begriff "heterolog" wird hier in seiner dem Fachmann bekannten Bedeutung verwendet, und bezieht sich auf die fremde Herkunft eines Elements, beispielsweise eines Enzyms oder anderen Proteins. "Fremd" bedeutet, dass das Element so in der Zielzelle nicht vorkommt, und beispielsweise aus einer Zelle oder einem Organismus mit abweichender genetischer Ausstattung, beispielsweise einem Organismus einer anderen Art, stammt.

Unter einem "Glycerolaufnahme-Facilitator-Protein" oder kurz "Glycerol-Facilitator" wird ein Transmembranprotein verstanden, das den Transport von Glycerol in eine Zelle, beispielsweie eine *Saccharomyces*-Zelle, mittels erleichterter Diffusion ermöglicht.

Unter "Glyceroldehydrogenase" (kurz GDH) wird hier eine cytosolische Glycerol:NAD⁺-Oxidoreduktase (EC 1.1.1.6) verstanden, welche die Oxidation von Glycerol zu Dihydroxyaceton mittels NAD⁺ als Kofaktor katalysiert. Unter einer "heterologen Glyceroldehydrogenase" wird eine cytosolische Fremd-Glyceroldehydrogenase verstanden, d.h. eine aus einem anderen Organismus, z.B. einer anderen Art, Gattung etc., stammende Glyceroldehydrogenase, die jedoch dieselbe Reaktion katalysiert, gegebenenfalls unter Nutzung eines anderen Kofaktors.

Unter dem Begriff "NADP-Glyceroldehydrogenase" (abgekürzt auch "NADP-GDH") wird hier eine Glycerol:NADP+-Oxidoreductase (EC 1.1.1.72) verstanden, die Glycerol zu D-Glycerinaldehyd oxidiert, wobei NADP+ als Kofaktor fungiert.

Unter dem Begriff "Dihydroxyacetonkinase" oder "DHA-Kinase" wird hier eine ATP:Dihydroxyaceton-Phosphotransferase (EC 2.7.1.29) verstanden, die die Phosphorylierung von Dihydroxaceton mittels ATP katalysiert.

Unter dem Begriff "Methanoldehydrogenase" (kurz MDH) wird hier ein die Reduktion von Formaldehyd zu Methanol (MeOH) katalysierendes Enzym (EC 1.1.1.244, EC 1.1.2.7, EC 1.1.2.8, 1.1.99.37) verstanden, vorzugsweise eine Methanol:NAD⁺-Oxidoreduktase (EC 1.1.1.244).

Unter einer "Dihydroxyaceton-Synthase" oder "DHA-Synthase", synonym auch als "Formaldehyd-Transketolase" oder "Transketolase" bezeichnet, wird eine D-Xylulose-5-phosphat:Formaldehyd-Glycolaldehydetransferase (EC 2.2.1.3) verstanden, die die Bildung von Formaldehyd und D-Xylulose-5-phosphat aus Dihydroxyaceton und Glycerinaldehyd-3-phosphat katalysiert.

Unter "Expression" wird hier die Umsetzung einer genetischen Information in ein Produkt verstanden, beispielsweise die Bildung eines Proteins oder einer Nukleinsäure anhand der genetischen Information. Der Begriff umfasst insbesondere die Biosynthese von Proteinen anhand der genetischen Information einschließlich vorangehender Prozesse wie der Transkription, d.h. der Bildung von mRNA auf Basis einer DNA-Vorlage.

Der Ausdruck, dass ein heterologes Protein/Enzym exprimiert wird, schließt ein, dass das Protein/Enzym im Vergleich zum durchschnittlichen Expressionsniveau des Proteins/Enzyms in der Herkunftszelle in der Zielzelle unter ansonsten gleichen Bedingungen überexprimiert wird. Der Ausdruck schließt auch ein, dass das Protein/Enzym unter Kontrolle eines beliebigen Promotors exprimiert wird, also auch eines Promotors, der von dem Promotor verschieden sein kann, der in der Herkunftszelle mit dem Protein/Enzym verknüpft ist oder der in der Zielzelle mit einem etwaigen orthologen Protein/Enzym verknüft ist. Dem Fachmann ist bekannt, wie er eine Überexpression eines Proteins/Enzyms bewirken kann. Eine Möglichkeit ist beispielsweise die Einführung zusätzlicher Kopien des für das Protein/Enzym kodierenden Gens in eine Zelle und/oder die Ersetzung des natürlichen Promotors, unter dessen Kontrolle die Expressin des Gens erfolgt, durch einen stärkeren Promotor.

Unter dem Begriff der "Blockade" oder des "Blockierens" eines Stoffwechselweges, d.h. einer Kette von Reaktionen mit einer Ausgangsverbindung, mindestens einem Zwischenprodukt und einem Endprodukt (das selbst auch wieder die Ausgangsverbindung für einen anderen Stoffwechselweg sein kann) wird hier verstanden, dass die Bildung zumindest des ersten Zwischenproduktes des Weges unterbunden oder wesentlich vermindert ist. "Blockade" oder "Blockieren" bedeutet hier insbesondere, dass im Vergleich zu einer Hefezelle, bei der der Stoffwechselweg nicht blockiert ist, die Bildung des ersten Zwischenprodukts um mindestens 75 %, vorzugsweise mindestens 80 %, mindestens 85 %, mindestens 90 %, mindestens 95 % oder mindestens 98 % vermindert ist. In Bezug auf den Glycerol-3-Phosphat-Stoffwechselweg wird unter einer Blockade daher verstanden, dass zumindest der Abbau von Glycerol zu Glycerol-3-Phosphat durch die Glycerolkinase blockiert ist. In Bezug auf den Syntheseweg zur Rückbildung von Glycerol aus Dihydroxyacetonphosphat (DHAP) wird unter einer Blockade verstanden, dass zumindest die Reduktion von DHAP zu Glycerol-3-Phosphat durch Glycerol-3-phosphat-Dehydrogenase oder zumindest die Reduktion von Glycerol-3-Phosphat zu Glycerol durch Glycerol-3-phosphatase blockiert ist. Der Begriff schließt auch ein, dass sämtliche Zwischenschritte auf dem Weg zum Endprodukt blockiert sind, beispielweise durch Deletieren oder Mutieren der Gene sämtlicher an dem Weg beteiligter Enzyme, so dass kein funktionsfähiges Genprodukt oder nur ein in seiner Funktion wesentlich eingeschränktes Genprodukt, d.h. Enzym, resultiert. Der Begriff kann auch bedeuten, dass die Bildung von funktionsfähigem Enzym um die oben genannten Prozentzahlen vermindert ist, indem beispielsweise die natürlichen Promotoren durch entsprechend schwächere ausgetauscht werden, oder dass nur eine solche Menge eines in seiner Funktionalität eingeschränkten Enzyms gebildet wird, dass die Bildung des Zwischenproduktes wie oben definiert vermindert ist. Mittel zur Modifizierung eines Stoffwechselweges im Wege des "Metabolic Engineering" sind dem Fachmann gut bekannt (s. z.B. Nevoigt 2008; Mapelli 2014). Beispielsweise kann ein Stoffwechselweg durch Deletieren oder Deaktivieren eines Gens oder eines für die Regulation eines Gens relevanten DNA-Abschnitts, beispielsweise mittels zielgerichteter Mutation, blockiert werden. Eine Modifikation dahingehend, dass ein heterologes Enzym exprimiert wird, kann durch dem Fachmann bekannte gentechnische Verfahren erreicht werden, beispielsweise indem ein geeigneter Expressionsvektor mit einem das Enzym kodierenden Gen (zusammen mit einem geeigneten Promoter und Terminator) in die Zelle eingebracht wird.

Wenn hier von einem Enzym mit einer bestimmten Enzymaktivität gesprochen wird, z.B. von einer Dihydroxyaceton-Kinase (DHA-Kinase), wird von dem Begriff auch ein Enzym erfasst, das neben mindestens einer anderen Enzymaktivität zumindest auch die entsprechende Enzymaktivität hat. Unter einer "DHA-Kinase" beispielsweise wird daher auch ein Enzym mit DHA-Kinase-Aktivität verstanden, dass daneben noch mindestens eine andere Enzymaktivität, beispielsweise eine Triosephosphatisomeraseaktivität, besitzt. Ein konkretes Beispiel hierfür ist die 2,3-Butandioldehydrogenase (Bdh1) aus *Saccharomyces cerevisiae*, von der gezeigt wurde, dass sie auch Glyceroldehydrogenaseaktivität aufweist (Gonzalez et al., 2000). Wenn hier von der Überexpression einer nativen Glyceroldehydrogenase gesprochen wird, umfasst dies daher auch beispielsweise die Überexpression der Bdh1 oder eines anderen nativen Enzyms mit entsprechender Aktivität.

Bei der gentechnisch veränderten Hefezelle gemäß der vorliegenden Erfindung handelt es sich vorzugsweise um eine Hefezelle der Art *Saccharomyces cerevisiae*.

In einer besonders bevorzugten Ausführungsform ist die erfindungsgemäße gentechnisch veränderte Hefezelle weiterhin dahingehend gentechnisch verändert, dass c1) der Abbau von Glycerol zu Glycerinaldehyd-3-Phosphat über den Glycerinaldehyd-Weg blockiert ist, und/oder
d) die Expression des durch *STL1* kodierten natürlichen aktiven Glycerol-Transporters blockiert ist, und/oder
e) der Syntheseweg zur Rückbildung von Glycerol aus Dihydroxyacetonphosphat ist blockiert ist.

Die Merkmale d), e) und c1) können einzeln oder in beliebiger Kombination oder auch alle zusammen mit den Merkmalen a), b), c) und f) verwirklicht sein. Merkmal c1) ist vorzugsweise nur zusammen mit den Merkmalen a), b), c) und f) verwirklicht, wobei zusätzlich die Merkmale d) und e) jeweils einzeln, in beliebiger Kombination oder kumuliert verwirklicht sein können.

Die zusätzliche Blockade des Glycerinaldehyd-Wegs ist beispielsweise für die Herstellung von Fermentationsprodukten vorteilhaft, für die Reduktionsäquivalente nur in Form von NADH benötigt werden, z.B. Succinat.

Durch die Blockade der Expression des natürlichen aktiven durch *STL1* kodierten Transporters wird ein unnötiger Verbrauch von ATP vermieden, insbesondere unter Bedingungen, bei denen die Zellen möglicherweise nicht genügend ATP liefern können, beispielsweise unter Sauerstofflimitierung. Die Expression des heterologen Glycerol-Facilitators ermöglicht dennoch die Aufnahme von Glycerol in die Hefezelle. Die Herstellung einer *STL1*-Deletionsmutante ist beispielsweise in Liu et al. 2013 und Swinnen et al. 2013 beschrieben.

Die Blockade des Synthesewegs zur Rückbildung von Glycerol aus Dihydroxyacetonphosphat verhindert zusätzlich, dass über den DHA-Weg gebildetes Dihydroxyacetonphosphat wieder zu Glycerol-3-Phosphat oder gar Glycerol umgesetzt wird, wodurch insbesondere ein unnötiger Abfluss von Reduktionsäquivalenten vermieden werden kann. Die Blockade kann beispielsweise durch Deletion der Gene (*GPD1*/*2* und/oder *GPP1*/*2*) erreicht werden, welche für die die Rückbildung katalysierenden Enzyme kodieren. Deletionen von *GPD1* bzw. *GPD2* sind in Hubmann et al. (2011) beschrieben. Die Deletion von *GPP1* bzw. *GPP2* ist in Pählmann et al. (2001) beschrieben. Alternativ können die natürlichen Promotoren durch sehr viel schwächere ausgetauscht werden, wie es für *GPD1* bzw. *GPD2* bereits beschrieben wurde (Hubmann et al., 2011). Anstelle einer Abschaltung von *GPD1*/*2* kann weiter alternativ auch eine Abschaltung von *GUT2* und damit eine Blockade der Expression der FAD-abhängigen mitochondrialen Glycerol-3-Phosphat-Dehydrogenase (mGDP) vorgenommen werden. Damit würde insbesondere bei aerober Anzucht der Verlust von Elektronen über das Glycerin-3-phosphat/DHAP-Shuttlesystem vermieden.

Die Expression einer heterologen DHA-Kinase kann anstatt oder zusätzlich zur nativen, d.h. natürlicherweise in der Zelle vorhandenen, DHA-Kinase erfolgen. Vorzugsweise wird eine heterologe DHA-Kinase mit einer im Vergleich zur nativen DHA-Kinase höheren Aktivität (höheren Umsatzrate) verwendet, oder die DHA-Kinase wird überexprimiert. In einer bevorzugten Ausführungsform der vorliegenden Erfindung ist die Hefezelle dahingehend gentechnisch verändert, dass der Abbau von Glycerol zu Dihydroxyacetonphosphat über den Glycerol-3-Phosphat-Weg dadurch blockiert ist, dass die Expression der Glycerolkinase und/oder der mitochondrialen Glycerol-3-Phosphat-Dehydrogenase (mGDP) blockiert ist. Bevorzugt ist, dass sowohl die Expression der Glycerolkinase als auch der mitochondrialen Glycerol-3-Phosphat-Dehydrogenase blockiert ist. Dies kann mit verschiedenen Methoden erreicht werden, die dem Fachmann bekannt sind, beispielsweise durch Deletion der für die Enzyme kodierenden Gene (s. z.B. Swinnen et al. 2013).

Bevorzugt handelt es sich bei dem heterologen Glycerolaufnahme-Facilitator-Protein um Fps1 oder Fps2, vorzugsweise Fps2 aus *Pachysolen tannophilus* (s. Liu et al. 2013; PtFPSl: GenBank: JQ481631.1 GI:394996125, AFN43530.1 GI:394996126; PtFPS2: JQ481632.1 GI:394996127, AFN43531.1 GI:394996128). Andere Proteine kommen jedoch ebenfalls in Frage, z.B. Fps1 aus *Pichia pastoris*, *Pichia jadinii* oder *Yarrowia lipolytica*. Bei der heterologen Glyceroldehydrogenase handelt es sich bevorzugt um eine Glyceroldehydrogenase aus *Ogataea angusta* (s. Jung et al. 2011, GenBank: AB185335.1 GI:50582511, BAD32688.1 GI:50582512). Anstelle der Einführung einer heterologen Glyceroldehydrogenase kann auch die in *Saccharomyces cerevisiae* natürlicherweise vorkommende und Glyceroldehydrogenaseaktivität aufweisende Bdh1 überexprimiert werden. Heterologe DHA-Kinasen stammen vorzugsweise aus Organismen, die nachweislich natürlicherweise den DHA-Weg benutzen, z.B. Dak1 aus *Schizosaccharomyces pombe* (s. Itoh et al., 1999, UniProtKB/Swiss-Prot 013902, Ver 98, 1106.2014.). Als heterologer Ersatz für die NADP⁺-abhängige Glyceroldehydrogenase des Glycerinaldehyd-Wegs kommt beispielsweise das Produkt des Gens gldB aus *Aspergillus* (*Emericella*) *nidulans* in Frage (de Vries et al. 2003, UniProtKB/Swiss-Prot Q7Z8L1, Ver 64, 11.06.2014). Als Ersatz für die Triosekinase kommt auch eine heterologe DHA-Kinase in Betracht. Alternativ oder zusätzlich können die für die Triosekinasen Dak1 oder Dak2 in *S*. *cerevisiae* kodierenden Gene *DAK1* und/oder *DAK2* überexprimiert werden.

Die erfindungsgemäße Hefezelle ist derart modifiziert, dass sie für die Herstellung reduzierter Fermentationsprodukte besonders geeignet ist. In der Ausgestaltung, in der der DHA-Weg aktiviert und/oder verstärkt ist, ist beispielsweise die Herstellung von Succinat besonders vorteilhaft realisierbar, weil hier die in der Redoxbilanz benötigten zwei NADH erzeugt werden. Je nach gewünschtem Fermentationsprodukt kann die Hefezelle entsprechend so manipuliert werden, dass die nötige Anzahl und Art von Reduktionsäquivalenten erzeugt wird. Dies schließt beispielsweise auch ein, dass der DHA-Weg in einer Weise aktiviert und/oder verstärkt wird, dass er NADPH liefert. Hierzu kann es vorteilhaft sein, noch weitere Veränderungen an der Hefezelle vorzunehmen.

So ist die Hefezelle in einer weiteren Ausführungsform der vorliegenden Erfindung dahingehend gentechnisch verändert, dass eine heterologe Formiatdehydrogenase (FDH) in der Hefezelle exprimiert oder die native Formiatdehydrogenase überexprimiert wird. Als heterologe Formiatdehydrogenasen kommen beispielsweise bakterielle Formiatdehydrogenasen, beispielsweise aus *E*. *coli*, in Frage. Bevorzugt sind jedoch Formiatdehydrogenasen eukaryotischen Ursprungs, beispielsweise aus *Candida* (z.B. *FDH1* aus *Candida boidinii*, UniProtKB/Swiss-Prot 013437 (FDH_CANBO), Ver 72, 16.04.14). Bei der überexprimierten Formiatdehydrogenase kann es sich auch um die vom *FDH1*-Gen kodierte Formiatdehydrogenase aus *Saccharomyces cerevisiae* handeln. Bei Kultivierung einer derart veränderten Hefezelle mit Formiat als Kosubstrat können zusätzliche Reduktionsäquivalente gewonnen werden, die beispielsweise bei Verwendung der Hefe zur Herstellung von insbesondere 1,2-Propandiol oder anderen stark reduzierten Produkten vorteilhaft sein können. Formiat (als Ko-Substrat verwendet) kann in diesem Zusammenhang als zusätzlicher Elektronendonator fungieren. Für den Fall, dass der Glycerinaldehyd-Weg durch Überexpression der relevanten Enzyme oder Einführung heterologer Varianten optimiert ist und daher NADPH als Reduktionsäquivalent anfällt, kann die 1,2-PDO Bildung auch mit NADPH betrieben werden, und alternativ zur Formatdehydrogenase könnte eine native Glucose-Dehydrogenase überexprimiert oder heterologe Glucose-Dehydrogenase eingeführt und Glucose als Co-Substrat eingesetzt werden.

In einer weiteren bevorzugten Ausführungsform ist die erfindungsgemäße Hefezelle darüber hinaus derart gentechnisch verändert, dass eine die Bildung von Formaldehyd aus Dihydroxyaceton katalysierende native Formaldehyd-Transketolase in der Hefezelle überexprimiert oder eine die Bildung von Formaldehyd aus Dihydroxyaceton katalysierende heterologe Formaldehyd-Transketolase in der Hefezelle exprimiert oder überexprimiert wird, und eine vorzugsweise NAD⁺-abhänige heterologe Methanoldehydrogenase (MDH) in der Hefezelle exprimiert oder überexprimiert wird. Diese Ausführungsform einer erfindungsgemäßen Hefezelle ist besonders geeignet für die Herstellung von Methanol aus Glycerol. Gegebenenfalls kann anstelle oder zusätzlich zu der (den) nativen Formaldehyd-Transketolase(n) eine heterologe Formaldehyd-Transketolase exprimiert werden.

Wie in der Beschreibungseinleitung bereits erwähnt, fällt Glycerol bei der Herstellung von Biodiesel an. Gleichzeitig wird hierbei in der Regel Methanol zur Umesterung der Fette und Öle eingesetzt. Es wäre daher vorteilhaft, Methanol in einem biotechnologischen Prozess aus dem anfallenden Glycerol zumindest teilweise zurückgewinnen zu können. Dies wird mit Hilfe dieser Ausführungsform einer erfindungsgemäß gentechnisch veränderten Hefezelle ermöglicht. Die Hefezelle ist dabei gentechnisch derart verändert, dass sie die folgende Umsetzung von Dihydroxyaceton (DHA) zu Methanol durchführt bzw. durchführen kann (GAP = Glycerinaldehyd-3-phosphat, XP5 = Xylulose-5-phosphat):

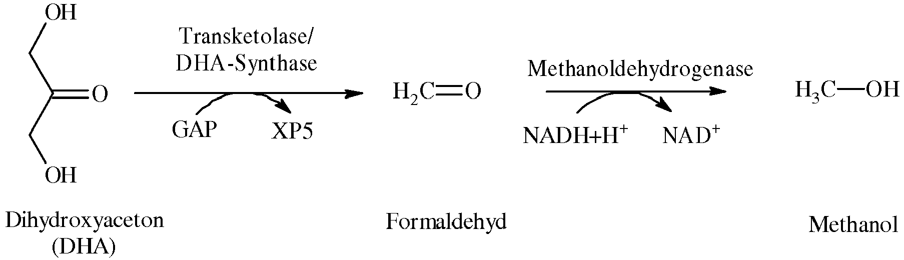

Während der erste Schritt, d.h. die Bildung von Formaldehyd aus DHA, in *Saccharomyces cerevisiae* natürlicherweise auch im Wildtyp auftritt (Molin und Blomberg 2006), und es daher bevorzugt ist, das oder die daran beteiligten Enzyme zu überexprimieren, findet der zweite Schritt mangels einer nativen Methanoldehydrogenase nur statt, wenn eine heterologe Methanoldehydrogenase in die Hefezelle eingeführt wird. Hierfür kommen insbesondere bakterielle Methanoldehydrogenasen in Frage. Methanoldehydrogenasen sind beispielsweise aus gram-negativen und gram-positiven methylotrophen Bakterien bekannt (Hanson & Hanson 1996; Duine et al. 1986). Ein Beispiel für eine geeignete Methanoldehydrogenase ist die NAD⁺-abhängige Methanoldehydrogenase aus dem gram-positiven Bakterium *Bacillus methanolicus* (Arfman et al. 1997).

Eine NAD⁺-abhängige Methanoldehydrogenase ist bevorzugt, weil auf diese Weise der Abbau von Glycerol zu Methanol hinsichtlich der verbrauchten und erzeugten Reduktionsäquivalente ausbalanciert ist. Das bei der Oxidation von Glycerol zu DHA reduzierte NAD⁺ wird bei der Reduktion des Formaldehyds zurückgewonnen.

Die Bäckerhefe verfügt über zwei homologe Formaldehyd-Transketolasen, die Transketolase 1 (Tkl1, YPR074C, s. http://www.yeastgenome.org/locus/S000006278/overview) und Transketolase 2 (Tkl2, YBR117C, s. http://www.yeastgenome.org/locus/S000000321/overview), um den ersten Schritt in der obigen Reaktionsgleichung zu katalysieren (Molin und Blomberg 2006). Die Überexpression eines oder beider Transketolasen kann in dem Fachmann bekannter Weise, beispielsweise durch Einführen weiterer Genkopien oder eines stärkeren Promotors, erfolgen.

Das für die Transketolase-Reaktion benötigte Glycerinaldehyd-3-phosphat (GAP) steht über den zentralen Kohlenstoffmetabolismus in der Hefe zur Verfügung. GAP kann beispielsweise auch erhalten werden, wenn DHA mittels der natürlicherweise in Hefe vorhandenen und von *DAK1* und *DAK2* codierten DHA-Kinase zu DHAP und mittels der von *TPI1* kodierten Triosephosphatisomerase (EC 5.3.1.1) weiter zu GAP umgesetzt wird. Dieser Weg ist besonders bevorzugt, wenn der Abbau von Glycerol zu Glycerinaldehyd-3-Phosphat über den Glycerinaldehyd-Weg blockiert ist.

Bei dieser Ausführungsform ist es besonders bevorzugt, wenn die Hefezelle ferner dahingehend gentechnisch verändert ist, dass die native DHA-Kinase gehemmt oder die Expression der nativen DHA-Kinase vermindert ist. Eine Verminderung der Genexpression der Gene *DAK1* und/oder *DAK2* kann beispielsweise durch Einführen eines schwächeren Promotors erfolgen (Nevoigt et al. 2006). Eine Verminderung der Aktivität der DHA-Kinase in der erfindungsgemäßen Hefezelle in geeigneter Weise und in geeignetem Ausmaß ermöglicht es, die Umsetzung von DHA so zu steuern, dass GAP-Bildung und Methanolbildung in einem für die Methanolherstellung geeigneten Verhältnis stehen.

In diesem Zusammenhang ist zu beachten, dass sowohl DHA als auch Formaldehyd zytotoxisch sind. Formaldehyd wird von *S*. *cerevisiae* daher zur Detoxifizierung über Formiat zu Kohlendioxid dissimliert (Molin und Blomberg 2006; Molin et al 2003), wobei die entsprechenden Schritte von einer durch *SFA1* codierten Glutathion-abhängigen Formaldehyddehydrogenase (EC 1.1.1.284) und einer durch *FDH1* codierten Formiatdehydrogenase (EC 1.2.1.2) katalysiert werden. In einer weiteren bevorzugten Ausführungsform ist die erfindungsgemäße Hefezelle daher dahingehend gentechnisch verändert, dass dieser Detoxifizierungsweg entweder blockiert ist oder, was bevorzugt ist, derart gentechnisch modifiziert ist, dass die Expression des SFA1-Gens vermindert oder ausgeschaltet wird, wenn der Formaldehydgehalt in der Zelle niedrig genug ist, um unerwünschte toxische Wirkungen in der Zelle hervorzurufen, oder alternativ die Expression des SFA1-Gens angeschaltet oder verstärkt wird, sobald der Formaldehydgehalt in der Zelle ein Niveau zu überschreiten droht, das unerwünschte toxische Wirkungen in der Zelle hervorufen kann. Gentechnische Verfahren, um diese Modikationen vorzunehmen, sind dem Fachmann bekannt (s. z.B. Borodina und Nielsen 2014). Alternativ oder gegebenenfalls zusätzlich kommt auch in Betracht, ein so genanntes "Metabolic Channelling" zu implementieren. Hierbei werden Enzyme, die aufeinanderfolgende Reaktion katalysieren, in einem gemeinsamen Komplex angeordnet, so dass verhindert wird, dass Zwischenprodukte in das Cytosol gelangen (s. Dueber, J.E. et al. 2009).

In einem weiteren Aspekt betrifft die vorliegende Erfindung auch ein Verfahren zur Herstellung eines Fermentationsproduktes aus Glycerol, wobei eine erfindungsgemäß gentechnisch veränderte Hefezelle nach dem oben beschriebenen Aspekt in einem glycerolhaltigen Kulturmedium unter geeigneten Fermentationsbedingungen kultiviert und das Fermentationsprodukt von dem Kulturmedium abgetrennt wird. Beispiele für Fermentationsprodukte sind Succinat, 1,2-Propandiol und Methanol. Im Fall von 1,2-Propandiol (1,2-PDO) ist es besonders bevorzugt, wenn die eingesetzte Hefezelle eine heterologe Formiatdehydrogenase und/oder eine native Formiatdehydrogenase enthält, die überexprimiert wird. Je nach gewünschtem Fermentationsprodukt kann eine Hefezelle eingesetzt werden, die für den jeweiligen Zweck insbesondere hinsichtlich der Redoxbilanz optimiert ist.

Die Erfindung wird im Folgenden anhand der beigefügten Figur beispielhaft näher erläutert.

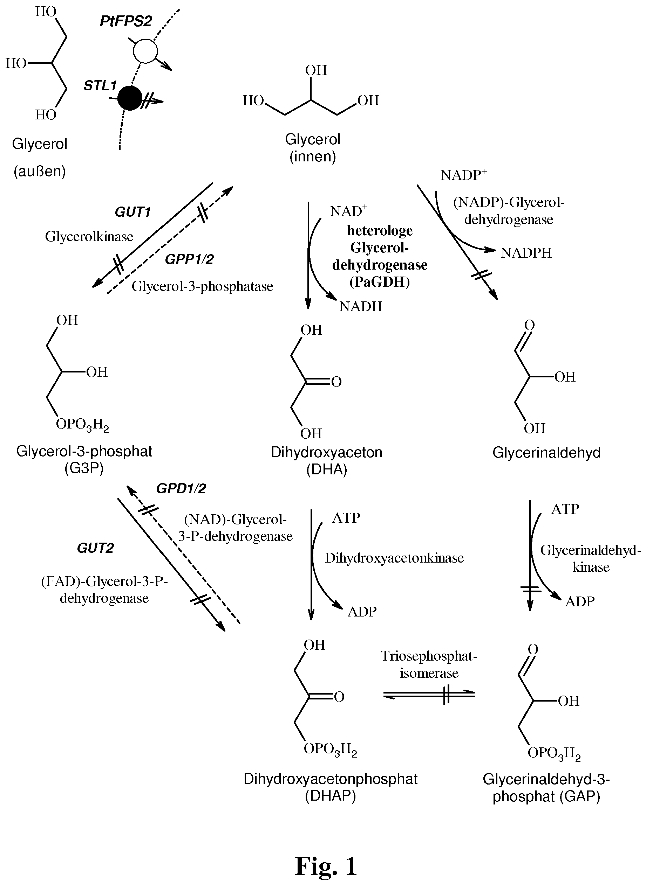

Fig. 1 Schematische Darstellung des Abbaus von Glycerol in einer Ausführungsform einer erfindungsgemäßen gentechnisch veränderten Hefezelle.
Fig. 1 zeigt schematisch den Abbau von Glycerol in einer bevorzugten Ausführungsform einer erfindungsgemäßen gentechnisch veränderten Hefezelle. Dargestellt sind der Glycerol-3-Phosphat-Weg (links), der DHA-Weg (Mitte) und der Glycerinaldehyd-Weg (rechts), also jene drei Stoffwechselwege, die grundsätzlich zu einem Glycerolabbau führen können. Bei der Hefezelle wurde die Expression des natürlichen Transportproteins Stl1 durch Deletion des entsprechenden Gens blockiert. Darüber hinaus wurde die Zelle dahingehend gentechnisch verändert, dass ein heterologer Glycerol-Facilitator, hier Fps2 aus *Pachysolen tannophilus*, exprimiert wird. Der Glycerol-3-Phosphat-Weg ist durch Deletion der Gene für Glycerolkinase (*GUT1*) und die mitochondriale Glycerol-3-Phosphat-Dehydrogenase (*GUT2*) blockiert. Darüber hinaus ist auch die Rückreaktion über die NAD-abhängige Glycerol-3-Phosphat-Dehydrogenase und die Glycerol-3-Phosphatase durch Deletion der hierfür kodierenden Gene (*GPD1*, *GPD2*, *GPP1*, *GPP2*) blockiert. In die Zelle über den Glycerol-Facilitator transportiertes Glycerol wird damit über den in der Mitte dargestellten DHA-Weg über Dihydroxyaceton zu Dihydroxyacetonphosphat (DHAP) umgesetzt. In der Hefezelle wird eine heterologe Glyceroldehydrogenase, hier eine Glyceroldehydrogenase aus *Ogataea angusta*, exprimiert. Des Weiteren ist vorzugsweise auch der Glycerinaldehyd-Weg bei dieser Ausführungsform vollständig durch Deletion der kodierenden Enzyme blockiert. Diese Ausführungsform ist besonders vorteilhaft für den Fall, dass Reduktionsäquivalente ausschließlich in Form von NADH benötigt werden, wie beispielsweise bei der Herstellung von Succinat. Die Bildung bzw. der Verbrauch von Reduktionsäquivalenten oder ATP ist nur für den DHA-Weg und den Glycerinaldehyd-Weg dargestellt.

### Referenzen

Almeida, J.R., Favaro, L.C., Quirino, B.F. 2012. Biodiesel biorefinery: opportunities and challenges for microbial production of fuels and chemicals from glycerol waste. Biotechnol Biofuels, 5(1), 48.

Arfman, N., Hektor, HJ., Bystrykh, L.V., Govorukhina, NI., Dijkhuizen, L., Frank, J. ( 1997. Properties of an NAD(H)-containing methanol dehydrogenase and its activator protein from Bacillus methanolicus. Eur. J. Biochem. 244: 426-433.

Babel, W., Hofmann, K.H. 1982. The relation between assimilation of methanol and glycerol in yeast. Arch Microbiol, 132, 179-184.

Borodina, I., Nielsen, Advances in metabolic engineering of yeast Saccharomyces cerevisiae for production of chemicals. J., Biotechnol J. 2014 May;9(5):609-620. doi: 10.1002/biot.201300445.

De Vries RP, Flitter SJ, van de Vondervoort PJ, Chaveroche MK, Fontaine T, Fillinger S, Ruijter GJ, d'Enfert C, Visser J. 2003. Glycerol dehydrogenase, encoded by gldB is essential for osmotolerance in Aspergillus nidulans. Mol Microbiol. 49(1):131-41. Dueber J.E., Wu G.C., Malmirchegini G.R., Moon T.S., Petzold C.J., Ullal A.V., Prather K.L., Keasling J.D. 2009. Synthetic protein scaffolds provide modular control over metabolic flux. Nat. Biotechnol. 27, 753-759

Duine, J.A., Frank Jzn., J., Jongejan J.A. 1986. PQQ and quinoprotein enzymes in microbial oxidations. FEMS Microbiol. Lett. 32: 165-178.

Fakas, S., Makri, A., Bellou, S., Aggelis, G. 2009. Pathways to aerobic glycerol catabolism and their regulation. in: Microbial conversions of raw glycerol, (Ed.) G. Aggelis, Nova Science Publishers, Inc. New York, pp. 9-18.

Ferreira, C., van Voorst, F., Martins, A., Neves, L., Oliveira, R., Kielland-Brandt, M.C., Lucas, C., Brandt, A. 2005. A member of the sugar transporter family, Stl1p is the glycerol/H+ symporter in Saccharomyces cerevisiae. Mol Biol Cell, 16(4), 2068-76. Gancedo, C., Gancedo, J.M., Sols, A. 1968. Glycerol metabolism in yeasts. Pathways of utilization and production. Eur J Biochem, 5(2), 165-72.

Gonzalez, E., Fernandez, M.R., Larroy, C., Sola, L., Pericas, M.A., Pares, X., Biosca, J.A. 2000. Characterization of a (2R,3R)-2,3-butanediol dehydrogenase as the Saccharomyces cerevisiae YAL060W gene product. Disruption and induction of the gene. J Biol Chem, 275(46), 35876-85.

Hanson, RS., Hanson, TE. 1996. Methanotrophic bacteria. Microbiol. Rev. 60: 439-471. Hofmann KH, Babel W (1979) Synthese und Akkumulation der Glycerin-Dehydrogenase in Candida valida H 122 in Abhängigkeit von der C-Quelle. VII. Reinhardsbrunner Symposium der Sektion Mikrobiologie der Biologischen Gesellschaft der DDR, vom 6. bis 12. Mai. In: Abhandl Akad Wiss DDR (N3):229-234. Berlin, Akademie-Verlag 1981 Hong, K.K., Nielsen, J. 2012. Metabolic engineering of Saccharomyces cerevisiae: a key cell factory platform for future biorefineries. Cell Mol Life Sci, 69(16), 2671-90. Hubmann, G, Guillouet, S., Nevoigt, E. 2011. Gpd1 and Gpd2 Fine-Tuning for Sustainable Reduction of Glycerol Formation in Saccharomyces cerevisiae. Appl. Env. Microbiol. 77, 5857-5867.

Itoh N, Tujibata Y, Liu JQ 1999. Cloning and overexpression in Escherichia coli of the gene encoding dihydroxyacetone kinase isoenzyme I from Schizosaccharomyces pombe, and its application to dihydroxyacetone phosphate production. Appl Microbiol Biotechnol. 51(2):193-200.

Izawa, S., Sato, M., Yokoigawa, K., Inoue, Y. 2004. Intracellular glycerol influences resistance to freeze stress in Saccharomyces cerevisiae: analysis of a quadruple mutant in glycerol dehydrogenase genes and glycerol-enriched cells. Appl Microbiol Biotechnol, 66(1), 108-14.

Jung, J.Y., Yun, H.S., Lee, J., Oh, M.K. 2011. Production of 1,2-propanediol from glycerol in Saccharomyces cerevisiae. J Microbiol Biotechnol, 21(8), 846-53.

Klingenberg, M. 1970. Localization of the glycerol-phosphate dehydrogenase in the outer phase of the mitochondrial inner membrane. Eur J Biochem, 13(2), 247-52.

Lin, E.C. 1976. Glycerol dissimilation and its regulation in bacteria. Annu Rev Microbiol, 30, 535-78.

Liu, X., Mortensen, U.H., Workman, M. 2013. Expression and functional studies of genes involved in transport and metabolism of glycerol in Pachysolen tannophilus. Microb Cell Fact, 12, 27.

Marktanalyse Nachwachsende Rohstoffe 2014, Schriftenreihe Nachwachsende Rohstoffe 34, Fachagentur Nachwachsende Rohstoffe e.V. (FNR)

May, JW, Sloan J., 1981. Glycerol utilization by Schizosaccharomyces pompedehydrogenation as the initial step. J Gen Microbiol 123:182-186

Merico, A., Ragni, E., Galafassi, S., Popolo, L., Compagno, C. 2011. Generation of an evolved Saccharomyces cerevisiae strain with a high freeze tolerance and an improved ability to grow on glycerol. J Ind Microbiol Biotechnol, 38(8), 1037-44.

Molin, M., Norbeck, J., Blomberg, A. 2003. Dihydroxyacetone kinases in Saccharomyces cerevisiae are involved in detoxification of dihydroxyacetone. J Biol Chem, 278(3), 1415-23.

Molin, M., Blomberg, A. 2006. Dihydroxyacetone detoxification in Saccharomyces cerevisiae involves formaldehyde dissimilation. Mol. Microbiol. 60: 925-938.

Neves, L., Lages, F., Lucas, C. 2004. New insights on glycerol transport in Saccharomyces cerevisiae. FEBS Lett, 565(1-3), 160-2.

Nevoigt, E., Kohnke, J., Fischer, CR., Alper, H., Stahl, U., Stephanopoulos, G. 2006. Engineering of promoter replacement cassettes for fine-tuning of gene expression in Saccharomyces cerevisiae. Appl. Environ. Microbiol. 72: 5266-5273

Nevoigt, E. 2008. Progress in metabolic engineering of Saccharomyces cerevisiae. Microbiol Mol Biol Rev, 72(3), 379-412.

Nguyen, H.T., Nevoigt, E. 2009. Engineering of Saccharomyces cerevisiae for the production of dihydroxyacetone (DHA) from sugars: a proof of concept. Metab Eng, 11(6), 335-46.

Norbeck, J., Blomberg, A. 1997. Metabolic and regulatory changes associated with growth of Saccharomyces cerevisiae in 1.4 M NaCl. Evidence for osmotic induction of glycerol dissimilation via the dihydroxyacetone pathway. J Biol Chem, 272(9), 5544-54.

Påhlman AK, Granath K, Ansell R, Hohmann S, Adler L. 2001. The yeast glycerol 3-phosphatases Gpplp and Gpp2p are required for glycerol biosynthesis and differentially involved in the cellular responses to osmotic, anaerobic, and oxidative stress. J Biol Chem 276(5):3555-563.

Shi, S., Valle-Rodriguez, J.O., Siewers, V., Nielson, J. 2011. Prospects for microbial biodiesel production. Biotechnol. J. 6: 277-285.

Sprague, G.F., Cronan, J.E. 1977. Isolation and characterization of Saccharomyces cerevisiae mutants defective in glycerol catabolism. J Bacteriol, 129(3), 1335-42. Swinnen, S., Klein, M., Carrillo, M., McInnes, J., Nguyen, H.T., Nevoigt, E. 2013. Reevaluation of glycerol utilization in Saccharomyces cerevisiae: characterization of an isolate that grows on glycerol without supporting supplements. Biotechnol Biofuels, 6(1), 157.

Tom GD, Viswanath-Reddy M, Howe Jr, Branch H (1978) Effect of carbon source on enzymes involved in glycerol metabolism in Neurospora crassa. Arch Microbiol 117: 259-263

Yazdani, S.S., Gonzalez, R. 2007. Anaerobic fermentation of glycerol: a path to economic viability for the biofuels industry. Curr Opin Biotechnol, 18(3), 213-9.

## Patentansprüche

1. Gentechnisch veränderte Hefezelle der Gattung *Saccharomyces* mit verbessertem Glycerol-Katabolismus, wobei die Hefezelle dahingehend gentechnisch verändert ist, dass
a) der Abbau von Glycerol zu Dihydroxyacetonphosphat über den Glycerol-3-Phosphat-Weg blockiert ist, und
b) ein heterologes Glycerolaufnahme-Facilitator-Protein exprimiert wird, und
c) eine die Oxidation von Glycerol zu Dihydroxyaceton katalysierende heterologe NAD-abhängige Glyceroldehydrogenase exprimiert oder eine die Oxidation von Glycerol zu Dihydroxyaceton katalysierende heterologe NADH-abhängige Glyceroldehydrogenase überexprimiert wird, und
f) eine heterologe Dihydroxyacetonkinase exprimiert oder die native Dihydroxyacetonkinase überexprimiert wird.

2. Gentechnisch veränderte Hefezelle nach Anspruch 1, wobei die Hefezelle eine Hefezelle der Art *Saccharomyces cerevisiae* ist.

3. Gentechnisch veränderte Hefezelle nach Anspruch 1 oder 2, wobei zusätzlich c1) der Abbau von Glycerol zu Glycerinaldehyd-3-Phosphat über den Glycerinaldehyd-Weg blockiert ist, und/oder
d) die Expression des durch *STL1* kodierten natürlichen aktiven Glycerol-Transporters blockiert ist, und/oder
e) der Syntheseweg zur Rückbildung von Glycerol aus Dihydroxyacetonphosphat blockiert ist.

4. Gentechnisch veränderte Hefezelle nach einem der vorhergehenden Ansprüche, wobei der Abbau von Glycerol zu Dihydroxyacetonphosphat über den Glycerol-3-Phosphat-Weg dadurch blockiert ist, dass die Expression der Glycerolkinase und/oder der mitochondrialen Glycerol-3-Phosphat-Dehydrogenase blockiert ist.

5. Gentechnisch veränderte Hefezelle nach einem der vorhergehenden Ansprüche, wobei das heterologe Glycerolaufnahme-Facilitator-Protein Fps1 oder Fps2 aus *Pachysolen tannophilus*, oder Fps1 aus *Pichia pastoris*, *Pichia jadinii* oder *Yarrowia lipolytica* ist, und die heterologe Glyceroldehydrogenase eine Glyceroldehydrogenase aus *Ogataea angusta* ist.

6. Gentechnisch veränderte Hefezelle nach einem der vorhergehenden Ansprüche, wobei die Hefezelle darüber hinaus dahingehend gentechnisch verändert ist, dass eine heterologe Formiatdehydrogenase in der Hefezelle exprimiert oder die native Formiatdehydrogenase überexprimiert wird.

7. Gentechnisch veränderte Hefezelle nach einem der Ansprüche 1 bis 5, wobei die Hefezelle darüber hinaus dahingehend gentechnisch verändert ist, dass eine die Bildung von Formaldehyd aus Dihydroxyaceton katalysierende native Formaldehyd-Transketolase in der Hefezelle überexprimiert oder eine die Bildung von Formaldehyd aus Dihydroxyaceton katalysierende heterologe Formaldehyd-Transketolase in der Hefezelle exprimiert oder überexprimiert wird, und eine vorzugsweise NAD⁺-abhänige heterologe Methanoldehydrogenase in der Hefezelle exprimiert oder überexprimiert wird.

8. Gentechnisch veränderte Hefezelle nach Anspruch 7, wobei die Hefezelle darüber hinaus dahingehend gentechnisch verändert ist, dass die native Dihydroxyacetonkinase gehemmt oder die Expression der nativen Dihydroxyacetonkinase vermindert ist.

9. Verfahren zur Herstellung eines Fermentationsproduktes aus Glycerol, wobei eine gentechnisch veränderte Hefezelle nach einem der vorhergehenden Ansprüche in einem glycerolhaltigen Kulturmedium unter geeigneten Fermentationsbedingungen kultiviert und das Fermentationsprodukt von dem Kulturmedium abgetrennt wird.

10. Verfahren nach Anspruch 9, wobei die Hefezelle eine gentechnisch veränderte Hefezelle nach Anspruch 6 ist und die Hefezelle mit Formiat als Kosubstrat kultiviert wird.

11. Verfahren nach Anspruch 9 oder 10, wobei das Fermentationsprodukt Succinat oder 1,2-Propandiol ist.

12. Verfahren nach Anspruch 9 oder10, wobei das Fermentationsprodukt Methanol ist.

## Claims

1. A genetically modified yeast cell of the genus *Saccharomyces* with improved glycerol catabolism, wherein the yeast cell is genetically modified to the effect that a) the degradation of glycerol into dihydroxyacetone phosphate through the glycerol-3-phosphate pathway is blocked, and
b) a heterologous glycerol uptake facilitator protein is expressed, and
c) a heterologous NAD-dependent glycerol dehydrogenase catalyzing the oxidation of glycerol into dihydroxyacetone is expressed, or a heterologous NADH-dependent glycerol dehydrogenase catalyzing the oxidation of glycerol into dihydroxyacetone is overexpressed, and
f) a heterologous dihydroxyacetone kinase is expressed or the native dihydroxyacetone kinase is overexpressed.

2. The genetically modified yeast cell according to claim 1, wherein the yeast cell is a yeast cell of the species *Saccharomyces cerevisiae*.

3. The genetically modified yeast cell according to claim 1 or 2, wherein additionally c1) the degradation of glycerol into glycerinaldehyde 3-phosphate through the glycerinaldehyde pathway is blocked, and/or
d) the expression of the natural active glycerol transporter coded by *STL1 I* is blocked, and/or
e) the synthesis pathway for converting dihydroxyacetone phosphate back into glycerol is blocked.

4. The genetically modified yeast cell according to one of the afore-mentioned claims,
wherein the degradation of glycerol into dihydroxyacetone phosphate through the glycerol-3-phosphate pathway is blocked by blocking the expression of the glycerol kinase and/or of the mitochondrial glycerol-3-phosphate dehydrogenase.

5. The genetically modified yeast cell according to one of the afore-mentioned claims,
wherein the heterologous glycerol uptake facilitator protein Fps1 or Fps2 comes from *Pachisolen tannophilus*, or Fps1 comes from *Pichia pastoris*, *Pichia jadinii* or *Yarrowia lipolytica*, and the heterologous glycerol dehydrogenase is a glycerol dehydrogenase from *Ogataea angusta*.

6. The genetically modified yeast cell according to one of the afore-mentioned claims,
wherein the yeast cell is additionally genetically modified to the effect that a heterologous formiate dehydrogenase is expressed in the yeast cell or that the native formiate dehydrogenase is overexpressed.

7. The genetically modified yeast cell according to one of the claims 1 to 5,
wherein the yeast cell is additionally genetically modified to the effect that a native formaldehyde transketolase catalyzing the formation of formaldehyde from dihydroxyacetone is overexpressed in the yeast cell or that a heterologous formaldehyde transketolase catalyzing the formation of formaldehyde from dihydroxyacetone is expressed or overexpressed in the yeast cell, and a preferably NAD⁺-dependent heterologous methanol dehydrogenase is expressed or overexpressed in the yeast cell.

8. The genetically modified yeast cell according to claim 7, wherein the yeast cell is additionally genetically modified to the effect that the native dihydroxyacetone kinase is inhibited or the expression of the native dihydroxyacetone kinase is reduced.

9. A method for producing a fermentation product from glycerol, wherein a genetically modified yeast cell according to one of the afore-mentioned claims is cultivated under appropriate fermentation conditions in a culture medium containing glycerol and the fermentation product is separated from the culture medium.

10. The method according to claim 9, wherein the yeast cell is a genetically modified yeast cell according to claim 6 and the yeast cell is cultivated with formiate as a co-substrate.

11. The method according to claim 9 or 10, wherein the fermentation product is succinate or 1,2-propanediol.

12. The method according to claim 9 or 10, wherein the fermentation product is methanol.

## Revendications

1. Cellule de levure du genre *Saccharomyces* génétiquement modifiée présentant un catabolisme amélioré, où la cellule de levure est modifiée génétiquement afin que :
a) la dégradation du glycérol en phosphate de dihydroxyacétone en passant par la voie du 3-phosphate de glycérol soit bloquée, et
b) une protéine hétérologue facilitatrice de l'absorption du glycérol soit exprimée, et
c) une glycérol déshydrogénase hétérologue NAD-dépendante catalysant l'oxydation de glycérol en dihydroxyacétone soit exprimée ou une glycérol déshydrogénase hétérologue NADH-dépendante catalysant l'oxydation de glycérol en dihydroxyacétone soit surexprimé, et
f) une dihydroxyacétone kinase hétérologue soit exprimée ou la dihydroxyacétone kinase native soit surexprimée.

2. Cellule de levure génétiquement modifiée selon la revendication 1, où la cellule de levure est une cellule de levure de l'espèce *Saccharomyces cerevisiae*.

3. Cellule de levure génétiquement modifiée selon la revendication 1 ou 2, où, en outre, c1) la dégradation de glycérol en glycéraldéhyde-3-phosphate par la voie glycéraldéhyde est bloquée, et/ou
d) l'expression du transporteur de glycérol actif naturel codé par le *STL1* est bloquée, et/ou
e) la voie de synthèse pour la reformation du glycérol à partir de la dihydroxyacétone phosphate est bloquée.

4. Cellule de levure génétiquement modifiée selon l'une des revendications précédentes, où la dégradation de glycérol en dihydroxyacétone phosphate par la voie du glycérol-3-phosphate est bloquée en bloquant l'expression de la glycérol kinase et/ou de la glycérol-3-phosphate déshydrogénase mitochondriale.

5. Cellule de levure génétiquement modifiée selon l'une des revendications précédentes, où la protéine facilitatrice de l'absorption de glycérol Fps1 ou Fps2 provient de *Pachysolen tannophilus*, ou Fps1 provient de *Pichia pastoris*, *Pichia jadinii* ou *Yarrowia lipolytica*, et la glycérol déshydrogénase hétérologue est une glycérol déshydrogénase provenant de *Ogataea angusta.*

6. Cellule de levure génétiquement modifiée selon l'une des revendications précédentes, où la cellule de levure est en outre génétiquement modifiée afin qu'une formiate déshydrogénase hétérologue soit exprimée dans la cellule de levure ou que la formiate déshydrogénase native soit surexprimée.

7. Cellule de levure génétiquement modifiée selon l'une des revendications 1 à 5, où la cellule de levure est en outre génétiquement modifiée afin qu'une formaldéhyde transcétolase native catalysant la formation de formaldéhyde à partir de dihydroxyacétone soit surexprimée dans la cellule de levure ou qu'une formaldéhyde transcétolase hétérologue catalysant la formation de formaldéhyde à partir de dihydroxyacétone soit exprimée ou surexprimée dans la cellule de levure, et qu'une méthanol déshydrogénase hétérologue de préférence NAD⁺-dépendante soit exprimée ou surexprimée dans la cellule de levure.

8. Cellule de levure génétiquement modifiée selon la revendication 7, où la cellule de levure est en outre génétiquement modifiée afin que la dihydroxacétone kinase native soit inhibée ou que l'expression de la dihydroxyacétone kinase native soit réduite.

9. Procédé de fabrication d'un produit de la fermentation du glycérol, où une cellule de levure génétiquement modifiée selon l'une des revendications précédentes est cultivée dans un milieu de culture contenant du glycérol dans des conditions de fermentation appropriées et le produit de la fermentation est séparé du milieu de culture.

10. Procédé selon la revendication 9, où la cellule de levure est une cellule de levure génétiquement modifiée selon la revendication 6 et la cellule de levure est cultivée avec du formiate en tant que co-substrat.

11. Procédé selon la revendication 9 ou 10, où le produit de la fermentation est du succinate ou du 1,2 propanediol.

12. Procédé selon la revendication 9 ou 10, où le produit de la fermentation est du méthanol.
